# EUROPÄISCHE PATENTSCHRIFT

(11) **EP 2 680 773 B1**
(45) Veröffentlichungstag und Bekanntmachung des Hinweises auf die Patenterteilung: **24.10.2018**
(21) Anmeldenummer: 12711359.5
(22) Anmeldetag: 02.03.2012
(51) Int. Cl.: A61B 17/88, B23D 29/02, B25B 7/12, B26B 17/02

(54) **ZANGE ZUM SCHNEIDEN VON WERKSTÜCKEN**
PLIERS FOR CUTTING WORKPIECES
TENAILLES À COUPER DES PIÈCES

(30) Priorität: 02.03.2011 DE 102011001013
(43) Veröffentlichungstag der Anmeldung: 08.01.2014
(73) Patentinhaber: Merz, Alexander, 78606 Seitingen (DE)
(72) Erfinder: Merz, Alexander, 78606 Seitingen (DE)
(74) Vertreter: Patentanwälte und Rechtsanwalt Weiß, Arat & Partner mbB
(86) Internationale Anmeldenummer: PCT/EP2012/053647
(87) Internationale Veröffentlichungsnummer: WO 2012/117096

(56) Entgegenhaltungen:
- AT-B- 17 406
- DE-A1- 3 302 875
- US-A- 401 308
- US-A- 3 716 879
- US-A1- 2008 000 091

## Beschreibung

Die Erfindung betrifft eine Zange zum Schneiden von Werkstücken insbesondere aus Metall mit zumindest einem Zangenschenkel und einem Druckelement, welche über einen Drehpunkt miteinander verbunden sind, wobei der Zangenschenkel ein Zangenmaul zur Aufnahme des Werkstücks ausbildet und dem Zangenschenkel ein Schneidelement mit einem Schneidmaul zugeordnet ist und das Schneidelement mittels einer Rasteinrichtung um einen Drehpunkt drehbar gegenüber dem Zangenschenkel angeordnet ist, wobei das Schneidelement gegenüberliegend von dem Schneidmaul eine Rastzahnung aufweist und die Rastzahnung mit einer Zahnleiste eines Druckstücks in Eingriff steht und das Druckstück bzw. die Zahnleiste mittels einem Kraftspeicher in Eingriff mit der Rastzahnung gehalten ist, wobei der Rastzahnung ein Feststeller mit einer weiteren Rastleiste zugeordnet ist.

### Stand der Technik

Zangen sind in vielfältiger Ausführung bekannt und auf dem Markt. Im vorliegenden Fall geht es vor allem um spezielle Zangen zum Schneiden von Werkstücken in der Unfallchirurgie. Sie werden z. B. in der Orthopädie zum Abklemmen von Drähten, Nägeln oder Schraubenelementen benötigt. Dabei ist die Verhinderung von Spänen od. dgl. Werkstoffpartikeln beim Trennvorgang zur Vermeidung von Infektionen eine hygienische Notwendigkeit. Bei der Drahtosteosynthese in der operativen Frakturbehandlung werden Knochenfragmente mit Drähten, meistens mit sogenannten "Kirschner"-Drähten miteinander verbunden. Zum Abschneiden dieser Drähte werden Seitenschneider oder ähnliche Werkzeuge verwendet, was aber ebenfalls die Gefahr von Werkstoffrückständen mit sich bringt. Zudem müssen erhebliche Kräfte auf die Zange aufgebracht werden, um den entsprechenden Draht zu schneiden.

Derartige medizinische bzw. chirurgische Zangen sind beispielsweise aus der EP 0 321 884 bekannt. Mit dieser Drahtzange wird ein Draht abgequetscht, was erhebliche Kraft erfordert. Ähnliches gilt auch für die Zange nach der DE 43 29 220 A1.

Eine Zange mit den Merkmalen des Oberbegriffs des Anspruchs 1 ist aus der DE 33 02 875 A1 bekannt.

### Aufgabe

Aufgabe der vorliegenden Erfindung ist es zum einem eine Zange der o. g. Art zu entwickeln, welche mit einer Hand zu bedienen ist und keinen sehr grossen Kraftaufwand benötigt, ferner aber zu keinem Werkstoffabtrag führt, der zurückbleiben kann.

### Lösung der Aufgabe

Zur Lösung der Aufgabe führt, dass sich das Druckstück mit einem Rundstück in einer Mulde in dem Druckelement abstützt und der Feststeller in dem Zangenschenkel verschiebbar angeordnet ist.

Diese Rasteinrichtung ist von besonderer Bedeutung. Durch ihre Betätigung wird der erforderliche Kraftaufwand wesentlich verringert. Die Betätigung der Rasteinrichtung erfolgt auch nicht kontinuierlich, sondern bevorzugt quasi durch ein Pumpen von Zangenschenkel bzw. Druckelement gegenüber dem: Zangenschenkel. In einem bevorzugten Beispiel wird vorgesehen, dass dem Zangenschenkel ein Schneidelement mit einem Schneidmaul zugeordnet ist, wobei ein Grund des Zangenmauls und ein Grund des Schneidmauls nahe einem Drehpunkt angeordnet sind, um welchen das Schneidelement in dem Zangenschenkel dreht.

Hierdurch wird der Draht nicht abgequetscht, sondern tatsächlich geschnitten. Das Schneidelement dreht sich um den Drehpunkt und schneidet dabei mit einer Schneide in das Werkstück ein, sodass dieses nach Vollendung der Drehung tatsächlich abgeschnitten ist. Zum einen wird dadurch vermieden, dass Späne od. dgl., entstehen zum anderen wird der Kraftaufwand reduziert.

In einem nicht beanspruchten Beispiel wird vorgesehen, dass dem Zangenschenkel ein Schneidelement mit einem Schneidmaul zugeordnet ist, wobei Zangenmaul und Schneidmaul in ihren Konturen aufeinander abgestimmt sind und das Schneidelement in einem Rollenkäfig in dem Zangenschenkel dreht.

Dies hat den Vorteil, dass ein geordnetes Zusammenwirken von Zangenmaul und Schneidmaul stattfindet. Das Schneidelement wird auf einem Grossteil seines Umfangs durch den Rollenkäfig gehalten und bei der Drehung um einen Drehpunkt gesichert geführt.

Gemäß der vorliegenden Erfindung weist das Schneidelement gegenüberliegend von dem Schneidmaul eine Rastzahnung auf. Diese Rastzahnung wirkt mit einer Zahnleiste eines Druckstücks zusammen, wobei dieses Druckstück von dem Druckelement betätigt wird. Hierzu stützt sich das Druckstück mit einem Rundstück in einer Mulde in dem Druckelement ab. Wird das Druckelement betätigt, nimmt das Druckstück mit der Zahnleiste, die in die Rastzahnung des Schneidelements eingreift, dieses Schneidelement mit und dreht es um den Drehpunkt. Hierdurch wird ein erforderlicher Kraftaufwand minimiert.

Damit das Druckstück bzw. die Zahnleiste in Eingriff mit der Rastzahnung verbleibt, ist dem Druckstück noch ein entsprechender Kraftspeicher zugeordnet, der so auf das Druckstück drückt, dass die Zahnleiste in Eingriff mit der Rastzahnung ist.

Um ein oben erwähntes Pumpen, das heisst, ein Nachführen des Druckstücks an der Rastzahnung zu ermöglichen, ist noch ein Feststeller vorgesehen, der das Schneidelement in der bereits erreichten Drehlage hält, wenn das Druckstück nachgeführt wird. Hierzu weist der Feststeller ebenfalls eine Rastleiste auf, die in die Rastzahnung des Schneidelements eingreift. Der Feststeller ist in dem Zangenschenkel verschiebbar angeordnet.

Das Druckelement ist bevorzugt schenkelartig ausgebildet und weist eine drehgelenkartige Verbindung mit dem Zangenschenkel auf. Hierzu ist an dem Druckelement ein Drehzapfen vorgesehen, der in einer Drehmulde in dem Zangenschenkel sitzt.

Damit ein Spreizen von Zangenschenkel und Druckelement verbessert wird, ist zwischen beiden ein entsprechender Kraftspeicher vorgesehen. Dabei kann es sich um einen federgelagerten Bolzen handeln.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
**Figur** 1 eine Draufsicht auf eine erfindungsgemässe Zange zum Schneiden von nicht näher gezeigten Werkstücken;
**Figur** 2 eine teilweise dargestellte Draufsicht auf einen Zangenschenkel der Zange nach Figur 1;
**Figur 3** eine verkleinert dargestellte Draufsicht auf ein Druckelement der Zange nach Figur 1;
**Figur 4** eine Draufsicht auf eine Abdeckung für die Zange nach Figur 1;
**Figur 5** eine verkleinert dargestellte Draufsicht auf ein Schneidelement für die Zange nach Figur 1;
**Figur 6** eine um 90° gedrehte Draufsicht auf das Schneidelement gemäss Figur 5;
**Figur 7** eine Seitenansicht eines Druckstücks aus der Zange nach Figur 1;
**Figur 8** eine Draufsicht auf das Druckstück gemäss Figur 7;
**Figur 9** eine Draufsicht auf einen Feststeller für die erfindungsgemässe Zange nach Figur 1;
**Figur 10** eine Seitenansicht des Feststellers gemäss Figur 9;
**Figur 11** eine Draufsicht auf die geöffnete Zange gemäss Figur 1 mit der Anordnung der einzelnen Bauteile.

Eine erfindungsgemässe Zange P zum Schneiden von nicht näher gezeigten Werkstücken weist einen Zangenschenkel 1 und ein dem Zangenschenkel 1 zugeordnetes schenkelartiges Druckelement 2 auf. Der Zangenschenkel 1 stützt sich in Schliesslage über einen Anschlag 3 gegen das Druckelement 2 ab. Dabei kann der Anschlag 3 elastisch ausgebildet sein.

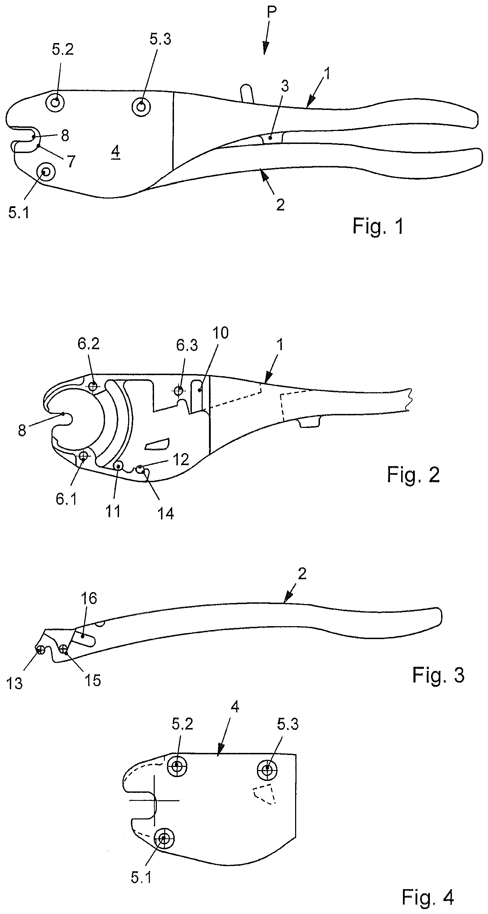

Im vorderen Bereich des Zangenschenkels 1 ist eine Abdeckung 4 zu erkennen, welche die wesentlichen Funktionselemente der Zange P verbirgt. Die Abdeckung 4 ist dabei über drei Schraubenbolzen 5.1 bis 5.3 mit dem Zangenschenkel 1 verbunden. Die Schraubenbolzen 5.1 bis 5.3 greifen in die Gewindebohrungen 6.1 bis 6.3 des Zangenschenkels 1 ein, wie sie in Figur 2 gezeigt sind.

Des Weiteren umrahmt die Abdeckung 4 mittels einer Ausnehmung 7 ein Zangenmaul 8, welches von dem Zangenschenkel 1 ausgebildet wird.

In Figur 2 ist ferner erkennbar, dass in dem Zangenschenkel 1 eine Führungsrinne 9 und ein Sackloch 10 vorgesehen sind. Des Weiteren sind Aufnahmen 11 und 12 ausgebildet. Die Aufnahme 11 dient der Lagerung eines später beschriebenen Anschlags, die Aufnahme 12 der Lagerung eines Drehzapfens 13 (siehe Figur 3), mit dem das Druckelement 2 in einer Drehmulde 14 in dem Zangenschenkel 1 sitzt. Neben diesem Drehzapfen 13 ist in dem Druckelement 2, gemäss Figur 3, eine Mulde 15 zur Aufnahme eines Rundstücks 22 eines später beschriebenen Druckstücks 21 ausgebildet. Zu dieser Mulde 15 hin ist ein weiteres Sackloch 16 offen.

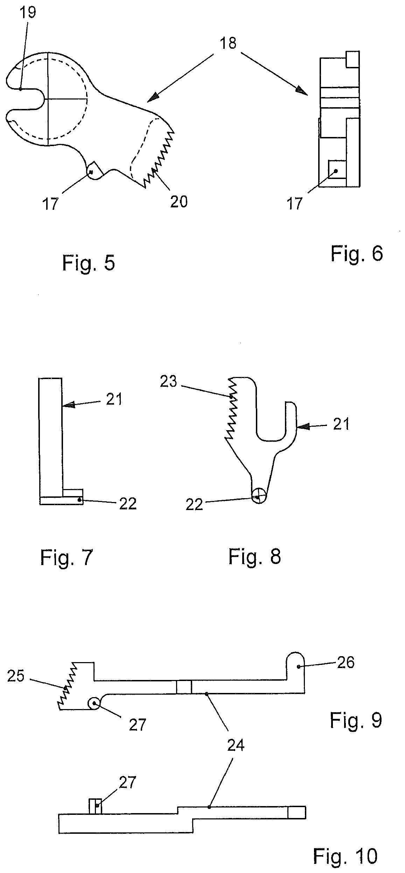

In die oben erwähnte Führungsrinne 9 greift ein Führungszapfen 17 ein, der gemäss Figur 5 und 6 von einem Schneidelement 18 abragt. Dieses Schneidelement 18 besitzt ein Schneidmaul 19, welches in seinen Konturen dem Zangenmaul 8 nachgeformt ist. Gegenüber dem Schneidmaul 19 ist an dem Schneidelement 18 eine Rastzahnung 20 vorgesehen.

Das oben erwähnte Druckstück 21, welches sich über ein Rundstück 22 in der Mulde 15 des Druckelementes 2 abstützt, ist in den Figuren 7 und 8 gezeigt. Dieses Druckstück 21 besitzt eine Zahnleiste 23. In den Figuren 9 und 10 wird ein Feststeller 24 gezeigt. Dieser Feststeller 24 weist eine Rastleiste 25 auf und dieser gegenüberliegend einen Zughacken 26. Nahe der Rastleiste 25 ist ferner ein Anschlagnocken 27 vorgesehen.

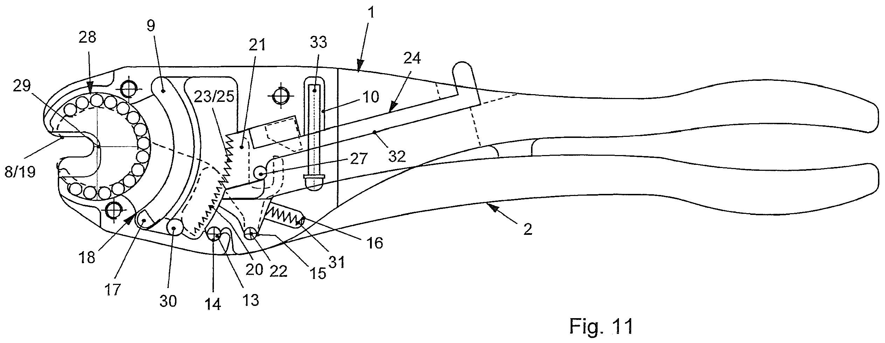

Das Zusammenspiel der einzelnen Bauteile ist insbesondere in Figur 11 erkennbar. Wesentlich ist dabei, dass das Schneidelement 18 im vorderen Bereich des Zangenschenkels 1 in einen Rollenkäfig 28 eingesetzt ist und in diesem Rollenkäfig 28 dreht. Diese Drehung erfolgt um einen Drehpunkt 29, wobei jeweils ein Grund des Zangenmauls 8 bzw. des Schneidmauls 19 sehr nahe an diesem Drehpunkt 29 angeordnet ist.

Im Übrigen ist erkennbar, dass das Schneidelement 18 mit seinem Führungszapfen 17 in die Führungsrinne 9 eingreift und in dieser Führungsrinne 9 in einem Radius um den Drehpunkt 29 geführt ist. Des Weiteren schlägt das Schneidelement 18 an einem in die Aufnahme 11 eingesetzten Anschlag 30 an.

Das Druckelement 2 sitzt mit seinem Drehzapfen 13 in der Drehmulde 14 und kann in begrenztem Umfang gegenüber dem Zangenschenkel 1 bewegt werden. In der Mulde 15 ist das Druckstück 21 mittels des Rundstücks 22 gelagert und stützt sich über eine Schraubenfeder 31 in dem Sackloch 16 ab. Die Zahnleiste 23 des Druckstücks 21 wirkt dabei mit der Rastzahnung 20 des Schneidelements 18 zusammen.

Der Festesteller 24 ist in einen Führungskanal 32 in dem Zangenschenkel 1 eingesetzt und in diesem Führungskanal 32 verschiebbar gelagert. Seine Rastleiste 25 überdeckt dabei zumindest teilweise die Zahnleiste 23 des Druckstücks 21. Der Anschlagnocken 27 bewirkt, dass der Feststeller 24 nicht unerwünscht nach hinten ausweichen kann.

Des Weiteren ist in Figur 11 ein Bolzen 33 angedeutet, der in das Sackloch 10 eingreift. Dieser Bolzen 33 kann federgelagert sein, sodass sich das Druckelement 2 gegenüber dem Zangenschenkel 1 federgelagert abstützt.

Die Funktionsweise der vorliegenden Erfindung ist folgende:
Zum Schneiden eines nicht näher gezeigten Werkstücks, wird dieses in das Zangen- bzw. Schneidmaul 8/19 eingeführt. Das Schneidelement 18 befindet sich dabei in der in Figur 11 gezeigten Ausgangsposition, sodass das Maul 8/19 weitestgehend geöffnet ist. Der Zangenschenkel 1 und das Druckelement 2 sind gespreizt, sodass die Zahnleiste 23 des Druckstücks 21 mit einer möglichst grossen Anzahl von Zähnen in Eingriff mit der Rastzahnung 20 des Schneidelements 18 steht. Dieser Eingriff wird durch Druck der Schraubenfeder 31 unterstützt.

Nun wird das Druckelement 2 zum Zangenschenkel 1 hin bewegt, wobei das Druckstück 21 das Schneidelement 18 mitnimmt, sodass dieses, geführt durch den Führungszapfen 17, in der Führungsrinne 9 und im Rollenkäfig 28 um den Drehpunkt 29 dreht. Dabei verringert sich die lichte Weite des Zangen- bzw. Schneidmauls 8/19, sodass das Schneidelement 18 in das Werkstück einschneidet. Gleichzeitig läuft auch die Rastzahnung 20 des Schneidelements 18 die Rastleiste 25 des Feststellers 24 ab und wird durch diesen Feststeller 24 gehalten, wenn nun zum Nachholen das Druckelement 2 wieder weg von dem Zangenschenkel 1 bewegt wird, sodass die Zahnleiste 23 des Druckstücks 21 entlang der Rastzahnung 20 fahren kann und in einen neuen Bereich dieser Rastzahnung 20 einfährt.

Jetzt kann erneut das Druckelement 2 wieder gegen den Zangenschenkel 1 bewegt werden, wobei das Druckstück 21 über die Rastverbindung mit dem Schneidelement 18 dieses mitnimmt und um den Drehpunkt 29 dreht. Dies geschieht so lange, bis das Werkstück durchgeschnitten ist.

**Bezugszeichenliste**

| | | | | | |
|---|---|---|---|---|---|
| 1 | Zangenschenkel | 34 | | 67 | |
| 2 | Druckelement | 35 | | 68 | |
| 3 | Anschlag | 36 | | 69 | |
| 4 | Abdeckung | 37 | | 70 | |
| 5 | Schraubenbolzen | 38 | | 71 | |
| 6 | Gewindebohrung | 39 | | 72 | |
| 7 | Ausnehmung | 40 | | 73 | |
| 8 | Zangenmaul | 41 | | 74 | |
| 9 | Führungsrinne | 42 | | 75 | |
| 10 | Sackloch | 43 | | 76 | |
| 11 | Aufnahme | 44 | | 77 | |
| 12 | Aufnahme | 45 | | 78 | |
| 13 | Drehzapfen | 46 | | 79 | |
| 14 | Drehmulde | 47 | | | |
| 15 | Mulde | 48 | | | |
| 16 | Sackloch | 49 | | | |
| 17 | Führungszapfen | 50 | | | |
| 18 | Schneidelement | 51 | | | |
| 19 | Schneidmaul | 52 | | | |
| 20 | Rastzahnung | 53 | | | |
| 21 | Druckstück | 54 | | | |
| 22 | Rundstück | 55 | | | |
| 23 | Zahnleiste | 56 | | | |
| 24 | Feststeller | 57 | | | |
| 25 | Rastleiste | 58 | | | |
| 26 | Zughaken | 59 | | | |
| 27 | Anschlagnocken | 60 | | P | Zange |
| 28 | Rollenkäfig | 61 | | | |
| 29 | Drehpunkt | 62 | | | |
| 30 | Anschlag | 63 | | | |
| 31 | Schraubenfeder | 64 | | | |
| 32 | Führungskanal | 65 | | | |
| 33 | Bolzen | 66 | | | |

## Patentansprüche

1. Zange zum Schneiden von Werkstücken im medizinischen bzw. chirurgischen Bereich insbesondere aus Metall mit zumindest einem Zangenschenkel (1) und einem Druckelement (2), welche über einen Drehpunkt (13) miteinander verbunden sind, wobei der Zangenschenkel (1) ein Zangenmaul (8) zur Aufnahme des Werkstücks ausbildet und dem Zangenschenkel (1) ein Schneidelement (18) mit einem Schneidmaul (19) zugeordnet ist und das Schneidelement (18) mittels einer Rasteinrichtung (20, 23, 25) um einen Drehpunkt (29) drehbar gegenüber dem Zangenschenkel (1) angeordnet ist, wobei das Schneidelement (18) gegenüberliegend von dem Schneidmaul (19) eine Rastzahnung (20) aufweist und die Rastzahnung (20) mit einer Zahnleiste (23) eines Druckstücks (21) in Eingriff steht und das Druckstück (21) bzw. die Zahnleiste (23) mittels einem Kraftspeicher (31) in Eingriff mit der Rastzahnung (20) gehalten ist, wobei der Rastzahnung (20) ein Feststeller (24) mit einer weiteren Rastleiste (25) zugeordnet ist, **dadurch gekennzeichnet, dass** sich das Druckstück (21) mit einem Rundstück (22) in einer Mulde (15) in dem Druckelement (2) abstützt und der Feststeller (24) in dem Zangenschenkel (1) verschiebbar angeordnet ist.

2. Zange nach Anspruch 1, **dadurch gekennzeichnet, dass** der Kraftspeicher (31) als Schraubenfeder in einem Sackloch (16) in dem Druckelement (2) gehalten ist.

3. Zange nach Anspruch 1 oder 2, **dadurch gekennzeichnet, dass** ein Grund des Zangenmauls (8) und ein Grund des Schneidmauls (19) nahe einem Drehpunkt (29) angeordnet sind, um welchen das Schneidelement (18) in dem Zangenschenkel (1) dreht und ausserhalb des Zangenmauls (8) und des Schneidmauls (19) angeordnet ist.

4. Zange nach wenigstens einem der Ansprüche 1 bis 3, **dadurch gekennzeichnet, dass** der Drehpunkt (13) als Drehzapfen (13) ausgebildet ist, das Druckelement (2) schenkelartig ausgebildet ist und mit dem Drehzapfen (13) in einer Drehmulde (14) in dem Zangenschenkel (1) sitzt.

5. Zange nach wenigstens einem der Ansprüche 1 bis 4, **dadurch gekennzeichnet, dass** sich das Druckelement (2) über einen Kraftspeicher gegen den Zangenschenkel (1) abstützt.

6. Zange nach Anspruch 5, **dadurch gekennzeichnet, dass** der Kraftspeicher ein federgelagerter Bolzen (33) ist.

## Claims

1. Pliers for cutting workpieces in the medical or surgical field, in particular made of metal with at least one pliers leg (1) and a pressure element (2) which are connected together via a pivot point (13), wherein the leg (1) of the pliers forms a pliers jaw (8) for receiving the workpiece, and a cutting element (18) with a cutting jaw (19) is associated with the leg (1) of the pliers, and the cutting element (18) is arranged so as to be rotatable about a pivot point (29) relative to the leg (1) of the pliers by means of a latching means (20, 23, 25), wherein the cutting element (18) has latching teeth (20) lying opposite the cutting jaw (19), and the latching teeth (20) are engaged with a toothed strip (23) of a pressure piece (21) and the pressure piece (21) or the toothed strip (23) is held in engagement with the latching teeth (20) by means of a force storage means (31), wherein an arresting means (24) with a further latching strip (25) is associated with the latching teeth (20), **characterised in that** the pressure piece (21) is supported with a round piece (22) in a depression (15) in the pressure element (2) and the arresting means (24) is arranged in the leg (1) of the pliers so as to be displaceable.

2. Pliers according to Claim 1, **characterised in that** the force storage means (31) is held as a helical spring in a blind hole (16) in the pressure element (2).

3. Pliers according to Claim 1 or 2, **characterised in that** a base of the jaw (8) of the pliers and a base of the cutting jaw (19) are arranged close to a pivot point (29) about which the cutting element (18) turns in the leg (1) of the pliers and is arranged outside the jaw (8) of the pliers and the cutting jaw (19).

4. Pliers according to at least one of Claims 1 to 3, **characterised in that** the pivot point (13) is designed as a pivot pin (13), the pressure element (2) is designed in the manner of a leg and is seated with the pivot pin (13) in a pivot depression (14) in the leg (1) of the pliers.

5. Pliers according to at least one of Claims 1 to 4, **characterised in that** the pressure element (2) is supported against the leg (1) of the pliers by means of a force storage means.

6. Pliers according to Claim 5, **characterised in that** the force storage means is a spring-mounted bolt (33).

## Revendications

1. Tenaille à couper des pièces dans le domaine médical et chirurgical, en particulier en métal avec au moins une branche de tenaille (1) et un élément de pression (2) qui sont reliés l'une à l'autre par l'intermédiaire d'un point de rotation (13), dans laquelle la branche de tenaille (1) forme une ouverture de tenaille (8) destinée à recevoir la pièce, et à la branche de tenaille (1) est associé un élément de coupe (18) avec une ouverture de coupe (19) et l'élément de coupe (18) est disposé au moyen d'un dispositif d'encliquetage (20, 23, 25) autour d'un point de rotation (29) de manière rotative par rapport à la branche de tenaille (1), dans laquelle l'élément de coupe (18) présente en regard de l'ouverture de coupe (19) une denture d'encliquetage (20) et la denture d'encliquetage (20) est en prise avec une barre d'encliquetage (23) d'une pièce de pression (21) et la pièce de pression (21) ou la barre d'encliquetage (23) est maintenue en prise, au moyen d'un accumulateur de force (31), avec la denture d'encliquetage (20), dans laquelle la denture d'encliquetage (20) est associée à un moyen d'arrêt (24) avec une autre barre d'encliquetage (25),
**caractérisée par le fait que** la pièce de pression (21) s'appuie par une pièce ronde (22) dans un creux (15) dans l'élément de pression (2) et le moyen d'arrêt (24) est disposé de manière déplaçable dans la branche de tenaille (1).

2. Tenaille selon la revendication 1, **caractérisée par le fait que** l'accumulateur de force (31) est maintenu comme ressort hélicoïdal dans un trou borgne (16) dans l'élément de pression (2).

3. Tenaille selon la revendication 1 ou 2, **caractérisée par le fait qu'**un fond de l'ouverture de tenaille (8) et un fond de l'ouverture de coupe (19) sont disposés à proximité d'un point de rotation (29) autour duquel l'élément de coupe (18) tourne dans la branche de tenaille et est disposé à l'extérieur l'ouverture de tenaille (8) et de l'ouverture de coupe (19).

4. Tenaille selon au moins l'une des revendications 1 à 3, **caractérisée par le fait que** le point de rotation (13) est réalisé sous forme de pivot (13), l'élément de pression (2) est réalisé en forme de branche et se trouve avec le pivot (13) dans un creux rotatif (14) dans la branche de tenaille (1).

5. Tenaille selon au moins l'une des revendications 1 à 4, **caractérisée par le fait que** l'élément de pression (2) s'appuie par l'intermédiaire d'un accumulateur de force contre la branche de tenaille (1).

6. Tenaille selon la revendication 5, **caractérisée par le fait que** l'accumulateur de force est un boulon à ressort (33).
